# EUROPEAN PATENT APPLICATION

(11) **EP 4 253 561 A1**
(43) Date of publication of application: **04.10.2023**
(21) Application number: 23164749.6
(22) Date of filing: 28.03.2023
(51) Int. Cl.: C12Q 1/682

(54) **METHOD FOR PRODUCING A COMPLEX, METHOD FOR DETERMINING MICROBIAL INCLUSION, AND METHOD FOR IDENTIFYING THE INCLUDED MICROORGANISM**

(30) Priority: 29.03.2022 JP 2022054622
(71) Applicant: Yokogawa Electric Corporation, Musashino-shi, Tokyo 180-8750 (JP)
(72) Inventor: TADENUMA, Takashi, Musashino-shi (JP); MIYAUCHI, Yuki, Musashino-shi (JP); TAGUCHI, Tomoyuki, Musashino-shi (JP); TANAAMI, Takeo, Musashino-shi (JP)
(74) Representative: Osha Liang

(57) **Abstract**

The present disclosure provides a method for producing a complex, a method for determining microbial inclusion, and a method for identifying the included microorganism, which can be easily performed. A method for producing a complex containing a target (40) that is contained in a sample comprises: a step (a) of bringing a sample into contact with a label molecule A (10), a label molecule B (30), and a label molecule C (20) and forming a complex; and a step (b) of isolating the complex. The target (40) is a nucleic acid., The label molecule A (10) contains a nucleic acid sequence capable of hybridizing to a part of the target (40) and substantially complementary to the nucleic acid sequence of the target (40). The label molecule B (30) contains a nucleic acid sequence capable of hybridizing to a part of the target (40) and substantially complementary to the nucleic acid sequence of the target (40).

## Description

### Technical Field

The present disclosure relates to a method for producing a complex, a method for determining microbial inclusion, and a method for identifying the included microorganism.

### Background Art

In the past, microbial testing of food, pharmaceutical, cosmetic, daily-use, and other products had been performed in accordance with, for example, the procedure shown in Fig. 1 or Fig. 2 (e.g., Patent Literature 1 (JP 2006-230335 A) and Patent Literature 2 (JP 2017-006012 A)). A conventional microbial testing procedure required approximately 4 steps: (1) nucleic acid (DNA or RNA) extraction; (2) nucleic acid amplification; (3) microarray detection; and (4) microbial (bacterial species) identification. In general, microarray detection was performed by fluorescence-labeling of a nucleic acid in a sample and hybridization of the labeled nucleic acid to a probe on microarrays. In the present disclosure, the term "hybridization" refers a situation in which at least parts of a nucleic acid molecule or a nucleic acid region of a molecule comprising a nucleic acid in a part of its molecular structure (e.g., a modified nucleic acid or a denatured nucleic acid) complementarily form a complex or an experiment or operation to form such complex.

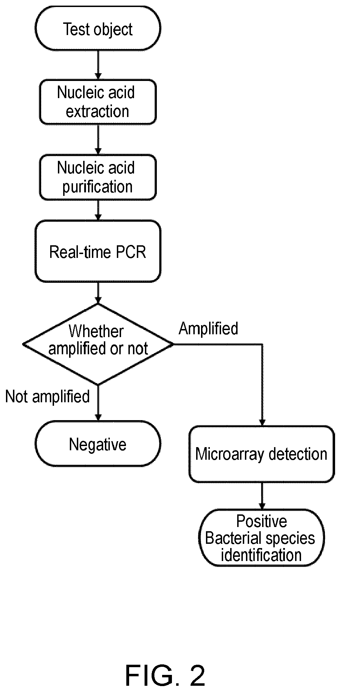

More specifically, DNA or RNA is extracted from bacteria that are present in the test object using a nucleic acid extraction kit. The nuclei acid is amplified by a polymerase chain reaction (PCR) or other means using the extracted nucleic acid (DNA or RNA) as a template. At the time of amplification, a target nucleic acid is fluorescence-labeled using a fluorescence-labeled primer, according to need. The amplified nucleic acid is applied to microarrays, such as DNA microarrays, comprising detection probes immobilized thereon to perform hybridization. After the completion of hybridization, the nucleic acid bound to the DNA microarrays is examined using a fluorometer. By performing such procedure, bacterial inclusion can be determined, and, when bacteria are included, the bacterial species can be determined (identified). At the time of nucleic acid amplification via PCR or other means, as shown in Fig. 2, the presence or absence of bacteria can be determined based on the results of amplification. In the presence of bacteria; i.e., when amplification is observed, the sample may be subjected to microarray detection, and the included bacteria may be identified.

### Citation List

### Patent Literature

Patent Literature 1 : JP 2006-230335 A
Patent Literature 2: JP 2017-006012 A

### Summary of Invention

### Technical Problem

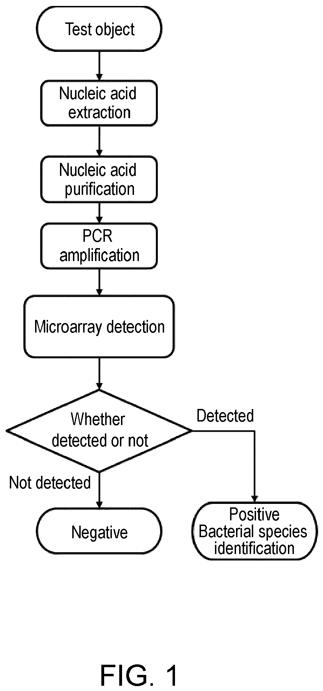

When microbial testing is performed in accordance with the procedure shown in Fig. 1, samples including no microorganisms (bacteria) are also subjected to microarray detection. This disadvantageously increases the cost for testing. When microbial testing is performed in accordance with the procedure shown in Fig. 2, approximately 1 to 2 hours are generally necessary to perform real-time PCR, the procedure is complicated due to the need of a step of purification for eliminating contaminants, and the cost for testing is further increased because of the use of an enzyme.

Accordingly, the present disclosure provides a method for producing a complex, a method for determining microbial inclusion, and a method for identifying the included microorganisms, which can be easily performed.

### Solution to Problem

We have conducted concentrated studies in order to provide such methods. As a result, we discovered that such methods would be provided by producing a complex comprising a target with the use of a plurality of particular label molecules. This has led to the completion of the present disclosure.

Embodiments of the present disclosure are defined in the claims and are described below.
[1] One or more embodiments are directed to a method for producing a complex comprising a target that is contained in a sample, the method comprising:
   a step (a) of bringing a sample containing the target into contact with a label molecule A, a label molecule B, and a label molecule C and forming a complex comprising the target, the label molecule A, the label molecule B, and the label molecule C; and
   a step (b) of isolating the complex,
   wherein
   the target is a nucleic acid,
   the label molecule A comprises a nucleic acid sequence capable of hybridizing to a part of the target and substantially complementary to a nucleic acid sequence of the target,
   the label molecule B comprises the nucleic acid sequence capable of hybridizing to a part of the target and substantially complementary to the nucleic acid sequence of the target,
   a substantially complementary nucleic acid sequence of the label molecule A is capable of hybridizing to a part of the target and a substantially complementary nucleic acid sequence of the label molecule B is capable of hybridizing to another part of the target,
   the label molecule C comprises a nucleic acid sequence capable of hybridizing to a part of the label molecule A and substantially complementary to the nucleic acid sequence of the label molecule A, and
   at least one of either the label molecule A or the label molecule C includes or has a phosphor.
[2] One or more embodiments are directed to the method for producing a complex according to [1], wherein the label molecule B includes or has at least one of either magnetic microparticles or metal microparticles.
[3] One or more embodiments are directed to the method for producing a complex according to [1], wherein the step (a) comprises:
   bringing the sample containing the target into contact with the label molecule A, the label molecule B, the label molecule C, and a label molecule D; and
   forming the complex comprising the target, the label molecule A, the label molecule B, the label molecule C, and the label molecule D,

   the label molecule D includes or has a nucleic acid sequence capable of hybridizing to a part of the label molecule B and substantially complementary to the nucleic acid sequence of the label molecule B, and
   at least one of either the label molecule B or the label molecule D includes or has at least one of either magnetic microparticles or metal microparticles.
[4] One or more embodiments are directed to the method for producing a complex according to any one of [1] to [3], wherein
   the label molecule A includes or has an x region, a y region, and a z region,
   the label molecule C includes or has an X region and a Y region,
   the x region is a nucleic acid sequence substantially complementary to a nucleic acid sequence of the X region,
   the y region is a nucleic acid sequence substantially complementary to a nucleic acid sequence of the Y region, and
   the z region is a nucleic acid sequence capable of hybridizing to a part of the target and substantially complementary to the nucleic acid sequence of the target.
[5] One or more embodiments are directed to the method for producing a complex according to [4], wherein
   the label molecule A includes or has two or more of at least either x regions or y regions in a molecule of the label molecule A and the label molecule C includes or has an X region and a Y region in a molecule of the label molecule C,
   the label molecule C includes or has two or more of at least either X regions or Y regions in a molecule of the label molecule C and the label molecule A includes or has an x region and a y region in a molecule of the label molecule A, or
   the label molecule A includes or has two or more of at least either x regions or y regions in a molecule of the label molecule A and the label molecule C includes or has two or more of at least either X regions and Y regions in a molecule of the label molecule C.
[6] One or more embodiments are directed to the method for producing a complex according to [3], wherein
   the label molecule B includes or has an u region, a v region, and a w region,
   the label molecule D includes or has an U region and a V region,
   the u region is a nucleic acid sequence substantially complementary to a nucleic acid sequence of the U region,
   the v region is a nucleic acid sequence substantially complementary to a nucleic acid sequence of the V region, and
   the w region is a nucleic acid sequence capable of hybridizing to a part of the target and substantially complementary to the nucleic acid sequence of the target.
[7] One or more embodiments are directed to the method for producing a complex according to [6], wherein
   the label molecule B includes or has two or more of at least either u regions or v regions in a molecule of the label molecule B and the label molecule D includes or has an U region and a V region in a molecule of the label molecule D,
   the label molecule D includes or has two or more of at least either U regions or V regions in a molecule of the label molecule D and the label molecule B includes or has an u region and a v region in a molecule of the label molecule B, or
   the label molecule B includes or has two or more of at least either u regions or v regions in a molecule of the label molecule B and the label molecule D includes or has two or more of at least either U regions or V regions in a molecule label molecule D.
[8] One or more embodiments are directed to a method for determining microbial inclusion in a test object, the method comprising:
   a step (1) of obtaining a sample by treatment of a test object under conditions in which a nucleic acid derived from a microorganism is extracted when the test object includes the microorganism;
   a step (2) of bringing the sample into contact with a label molecule A, a label molecule B, and a label molecule C and, when the sample contains a nucleic acid derived from the microorganism, forming a complex comprising a target, the label molecule A, the label molecule B, and the label molecule C; and
   a step (3) of isolating the complex when the complex is formed,
   wherein
   the target is a nucleic acid derived from the microorganism,
   the label molecule A comprises a nucleic acid sequence capable of hybridizing to a part of the target and substantially complementary to a nucleic acid sequence of the target,
   the label molecule B comprises the nucleic acid sequence capable of hybridizing to a part of the target and substantially complementary to the nucleic acid sequence of the target,
   a substantially complementary nucleic acid sequence of the label molecule A is capable of hybridizing to a part of the target and a substantially complementary nucleic acid sequence of the label molecule B is capable of hybridizing to another part of the target,
   the label molecule C comprises a nucleic acid sequence capable of hybridizing to a part of the label molecule A and substantially complementary to the nucleic acid sequence of the label molecule A, and
   at least one of either the label molecule A or the label molecule C includes or has a phosphor.
[9] One or more embodiments are directed to the method for determining microbial inclusion according to [8], wherein the label molecule B includes or has at least one of either magnetic microparticles or metal microparticles.
[10] One or more embodiments are directed to the method for determining microbial inclusion according to [8], wherein
   the step (2) comprises bringing the sample into contact with the label molecule A, the label molecule B, the label molecule C, and a label molecule D and, when the sample contains the nucleic acid derived from the microorganism, forming a complex comprising the target, the label molecule A, the label molecule B, the label molecule C, and the label molecule D,
   the label molecule D includes or has a nucleic acid sequence capable of hybridizing to a part of the label molecule B and substantially complementary to the nucleic acid sequence of the label molecule B, and
   at least one of either the label molecule B or the label molecule D includes or has at least one of either magnetic microparticles or metal microparticles.
[11] One or more embodiments are directed to the method for determining microbial inclusion according to any one of [8] to [10], wherein the step (1) comprises:
   a step of introducing the test object into a container;
   a step of hermetically sealing the container; and
   a step of heating the test object hermetically sealed in the container to 100°C or higher in a hermetically sealed state.
[12] One or more embodiments are directed to a method for identifying a microorganism included in a test object, the method comprising:
   a step (2) of bringing a sample derived from the test object into contact with a label molecule A, a label molecule B, and a label molecule C and, when the sample contains a nucleic acid derived from the microorganism, forming a complex comprising a target, the label molecule A, the label molecule B, and the label molecule C;
   a step (3) of isolating the complex when the complex is formed; and
   a step (4) of identifying the microorganism from which a nucleic acid sequence of the target contained in the complex is derived,
   wherein
   the target is a nucleic acid derived from the microorganism,
   the label molecule A comprises a nucleic acid sequence capable of hybridizing to a part of the target and substantially complementary to the nucleic acid sequence of the target,
   the label molecule B comprises a nucleic acid sequence capable of hybridizing to a part of the target and substantially complementary to the nucleic acid sequence of the target,
   a substantially complementary nucleic acid sequence of the label molecule A is capable of hybridizing to a part of the target and a substantially complementary nucleic acid sequence of the label molecule B is capable of hybridizing to another part of the target,
   the label molecule C comprises a nucleic acid sequence capable of hybridizing to a part of the label molecule A and substantially complementary to the nucleic acid sequence of the label molecule A, and
   at least one of either the label molecule A or the label molecule C includes or has a phosphor.
[13] One or more embodiments are directed to the method for identifying the included microorganism according to [12], wherein the label molecule B includes or has at least one of either magnetic microparticles or metal microparticles.
[14] One or more embodiments are directed to the method for identifying the included microorganism according to [12], wherein
   the step (2) comprises bringing a sample derived from a test object into contact with the label molecule A, the label molecule B, the label molecule C, and a label molecule D and, when the sample contains a nucleic acid derived from the microorganism, forming a complex comprising the target, the label molecule A, the label molecule B, the label molecule C, and the label molecule D,
   the label molecule D includes or has a nucleic acid sequence capable of hybridizing to a part of the label molecule B and substantially complementary to the nucleic acid sequence of the label molecule B, and
   at least one of either the label molecule B or the label molecule D includes or has at least one of either magnetic microparticles or metal microparticles.
[15] One or more embodiments are directed to the method for identifying the included microorganism according to any of [12] to [14], which comprises, before the step (2):
   a step (1) of obtaining a sample by treatment of the test object under conditions in which the nucleic acid derived from the microorganism is extracted when the test object includes the microorganism.

### Advantageous Effects of Invention

The present disclosure provides a method for producing a complex, a method for determining microbial inclusion, and a method for identifying the included microorganisms, which can be easily performed.

### Brief Description of the Drawings

Fig. 1 shows a flow chart demonstrating a procedure according to an embodiment of a conventional microbial testing technique.
Fig. 2 shows a flow chart demonstrating a procedure according to another embodiment of a conventional microbial testing technique.
Fig. 3 schematically shows a label molecule A which may be used in one or more embodiments of the present disclosure.
Fig. 4 schematically shows a label molecule C which may be used in one or more embodiments of the present disclosure.
Fig. 5 schematically shows a label molecule B which may be used in one or more embodiments of the present disclosure.
Fig. 6 schematically shows a complex which may be formed in one or more embodiments of the present disclosure.
Fig. 7 shows a flow chart demonstrating a procedure according to one or more embodiments of the method for identifying microorganisms according to the present disclosure.
Fig. 8 shows sequences of the target DNA, the label molecule A, the label molecule C, and the label molecule B used in the examples.

### Description of Embodiments

Hereafter, the method for producing a complex, the method for determining microbial inclusion, and the method for identifying the included microorganism according to one or more embodiments of the present disclosure are described in detail. The method for producing a complex according to an aspect of the present disclosure comprises producing a complex comprising a target. The target is a nucleic acid. The method for producing a complex is also performed in a part of the method for determining microbial inclusion and the method for identifying the included microorganism.

### (The method for producing a complex)

The method for producing a complex according to one or more embodiments of the present disclosure comprises producing a complex comprising a target that is contained in a sample, and the method comprises:
a step (a) of bringing a sample containing the target into contact with a label molecule A, a label molecule B, and a label molecule C and forming a complex comprising the target, the label molecule A, the label molecule B, and the label molecule C; and
a step (b) of isolating the complex.

The target is a nucleic acid, and, in general, such nucleic acid comprises a particular sequence. The label molecule A comprises a nucleic acid sequence capable of hybridizing to a part of the target and substantially complementary to a nucleic acid sequence of the target. The label molecule B comprises the nucleic acid sequence capable of hybridizing to a part of the target and substantially complementary to the nucleic acid sequence of the target. A substantially complementary nucleic acid sequence of the label molecule A is capable of hybridizing to a part of the target and a substantially complementary nucleic acid sequence of the label molecule B is capable of hybridizing to another part of the target. The label molecule C comprises a nucleic acid sequence capable of hybridizing to a part of the label molecule A and substantially complementary to the nucleic acid sequence of the label molecule A. At least one of either the label molecule A or the label molecule C includes or has a phosphor.

A sample is a component used in the method for producing a complex. In general, a sample is a component that is obtained by treatment of a test object. When a microorganism is included in a test object, for example, a sample is obtained by treatment of the test object under conditions in which a nucleic acid derived from the microorganism is extracted. As the conditions in which a nucleic acid is extracted, conventional conditions can be employed. For example, a technique similar to the treatment that had been performed as a pre-treatment of PCR may be employed. Nucleic acid extraction may be performed with the use of a commercially available extraction kit.

In general, the target is a nucleic acid comprising a particular sequence, which may be DNA or RNA. In general, the target is a nucleic acid derived from a microorganism. Examples of microorganisms include, but are not particularly limited to, bacteria, algae, slime molds, and viruses. A particular sequence can be adequately determined in accordance with, for example, a target microorganism to be detected.

As described above, the label molecule A comprises a nucleic acid sequence capable of hybridizing to a part of the target and substantially complementary to a nucleic acid sequence of the target, and the label molecule B comprises the nucleic acid sequence capable of hybridizing to a part of the target and substantially complementary to the nucleic acid sequence of the target. A substantially complementary nucleic acid sequence of the label molecule A is capable of hybridizing to a part of the target and a substantially complementary nucleic acid sequence of the label molecule B is capable of hybridizing to another part of the target. The label molecule C comprises a nucleic acid sequence capable of hybridizing to a part of the label molecule A and substantially complementary to the nucleic acid sequence of the label molecule A. Specifically, the complex comprises a target, a label molecule A hybridized to a part of the target, a label molecule B hybridized to another part of the target, and a label molecule C hybridized to a part of the label molecule A.

The term "substantially complementary nucleic acid sequence" used herein refers to both a nucleic acid sequence that is completely complementary to a given sequence and a nucleic acid sequence having mismatching, deletion, or addition of one or more nucleotides, provided that it can hybridize to a given sequence. In one or more embodiments, the number of mismatched nucleotides in a nucleic acid sequence may be 5 or fewer, 2 or fewer, or 1 or fewer. In one or more embodiments, the number of deleted or added nucleotides in a nucleic acid sequence may be 5 or fewer, 2 or fewer, or 1 or fewer.

At least one of either the label molecule A or the label molecule C includes or has a phosphor. In one or more embodiments, both the label molecule A and the label molecule C may include or have phosphors. In one or more embodiments, a phosphor may be in any position in the label molecule A and the label molecule C, and a phosphor may be positioned at the molecular end (e.g., the 5' end or the 3' end). Specifically, the label molecule A may include or have a phosphor at the molecular end and the label molecule C may include or have a phosphor at the molecular end.

A phosphor is not particularly limited, provided that it can emit fluorescence. In one or more embodiments, the excitation wavelength may be in the ultraviolet range (e.g., 280 to 380 nm) or in the visible light range (e.g., 380 to 830 nm). In one or more embodiments, a phosphor may have the emission wavelength in the visible light range (e.g., 380 to 830 nm). In one or more embodiments, the emission wavelength may be shifted to the longer wavelength side from the excitation wavelength by 15 nm or more. In one or more embodiments, the emission wavelength may be shifted by 20 to 100 nm or more.

Phosphors are not particularly limited. For example, organic fluorescent molecules, inorganic phosphors, quantum dots, and phosphor-integrated nanoparticles comprising a plurality of phosphors, such as organic fluorescent molecules, inorganic phosphors, and quantum dots, can be used. Examples of organic fluorescent molecules include EDANS, Coumarin, FAM, FITC, Cy2, TF2, TF3, HEX, JOE, TET, Cy3, Cy5, Alexa Fluor^{®} 532, Alexa Fluor^{®} 610, Alexa Fluor^{®} 647, ATTO532, ATTO633, iFluor 532, and iFluor 647. Examples of quantum dots include Qdot^{®} 565, Qdot^{®} 585, Qdot^{®} 605, and Qdot^{®} 705. Alternatively, phosphors (e.g., organic fluorescent molecules, inorganic phosphors, or quantum dots) supported on resin (e.g., polyolefin, polystyrene, or acrylic resin) or glass may be used.

In one or more embodiments, the label molecule A may comprise an x region, a y region, and a z region, the label molecule C may comprise an X region and a Y region, the x region may comprise a nucleic acid sequence substantially complementary to the nucleic acid sequence of the X region, the y region may comprise a nucleic acid sequence substantially complementary to the nucleic acid sequence of the Y region, and the z region may comprise a nucleic acid sequence capable of hybridizing to a part of the target and substantially complementary to the nucleic acid sequence of the target. The label molecule A may comprise a nucleic acid sequence other than the nucleic acid sequence of the x region, the y region, or the z region, and the label molecule C may comprise a nucleic acid sequence other than the nucleic acid sequence of the X region or the Y region.

Examples of specific combinations of the label molecule A and the label molecule C include the following embodiments: one or more embodiments in which the label molecule A has two or more of at least either x regions or y regions in its molecule and the label molecule C has an X region and a Y region in its molecule; one or more embodiments in which the label molecule C has two or more of at least either X regions and Y regions in a molecule of and the label molecule A has an x region and a y region in its molecule; and one or more embodiments in which the label molecule A has two or more of at least either x regions or y regions in its molecule and the label molecule C has two or more of at least either X regions and Y regions in its molecule. According to such embodiments, the complex can comprise a plurality of label molecules A and label molecules C. This improves the brightness in fluorescence observation.

In one or more embodiments, the label molecule A may have an x region and 2 y regions in its molecule, and the label molecule C may have 2 X regions and a Y region in its molecule.

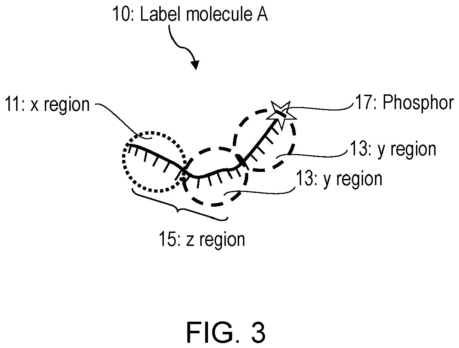

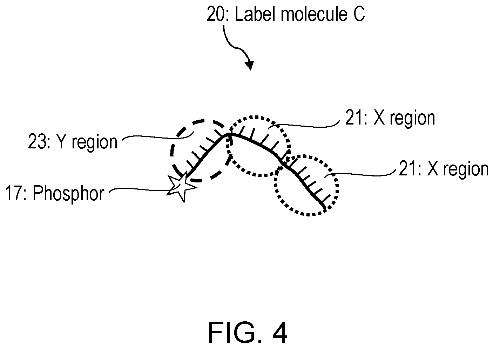

In one or more embodiments, the x region may be located closer to the 5' end than the y region in the label molecule A, and the X region may be located closer to the 5' end than the Y region in the label molecule C. In one or more embodiments, the label molecule A may comprise nucleic acid sequences of the x region, the (first) y region, and the (second) y region from the 5' end, and the label molecule C may comprise nucleic acid sequences of the (first) X region, the (second) X region, and the y region from the 5' end. In the label molecule A, the nucleic acid sequence of the z region may be different from that of the x region or the y region, and the nucleic acid sequence of at least either the x region or the y region may comprise the nucleic acid sequence of the z region. Fig. 3 schematically shows a label molecule A which may be used in one or more embodiments of the present disclosure. The label molecule A shown in Fig. 3 comprises nucleic acid sequences of the x region, the (first) y region, and the (second) y region from the 5' end and a phosphor at the 3' end. The z region is located across a part of the x region and a part of the (first) y region. Fig. 4 schematically shows a label molecule C which may be used in one or more embodiments of the present disclosure. The label molecule C shown in Fig. 4 comprises nucleic acid sequences of the (first) X region, the (second) X region, and the Y region from the 5' end and a phosphor at the 3' end. When the label molecule A shown in Fig. 3 hybridizes to a target, it is used for hybridization to the targets in the x region and the (first) y region. Since the label molecule A has the (second) y region, the Y region of the label molecule C may be able to hybridize to the (second) y region. Upon further hybridization of another label molecule A to the label molecule C, a plurality of label molecules A and label molecule C would be contained in the complex.

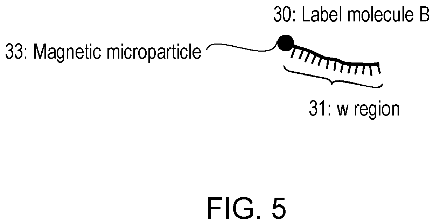

In one or more embodiments, the label molecule B may have at least one of either magnetic microparticles or metal microparticles. In some other embodiments, the label molecule B may have magnetic microparticles. Magnetic microparticles are not particularly limited, provided that such microparticles can be collected with the aid of a magnet. Metal microparticles are particles other than the magnetic microparticles, and any metal microparticles may be used without particular limitation, provided that such particles can be collected via precipitation because of high specific gravity. At least one of either magnetic microparticles or metal microparticles may be located in any positions in the label molecule B. In one or more embodiments, microparticles may be located at the molecular end (e.g., at the 5' end or the 3' end). In one or more embodiments, specifically, the label molecule B may comprise at least one of either magnetic microparticles or metal microparticles at the molecular end. In some other embodiments, the label molecule B may comprise magnetic microparticles at the molecular end. The label molecule B comprises a nucleic acid sequence capable of hybridizing to a part of a target and substantially complementary to the nucleic acid sequence of the target. Such nucleic acid sequence is also referred to as a "w region." The label molecule B may comprise a nucleic acid sequence other than that of the w region. Fig. 5 schematically shows a label molecule B which may be used in one or more embodiments of the present disclosure. The label molecule B schematically shown in Fig. 5 comprises magnetic microparticles at the 5' end.

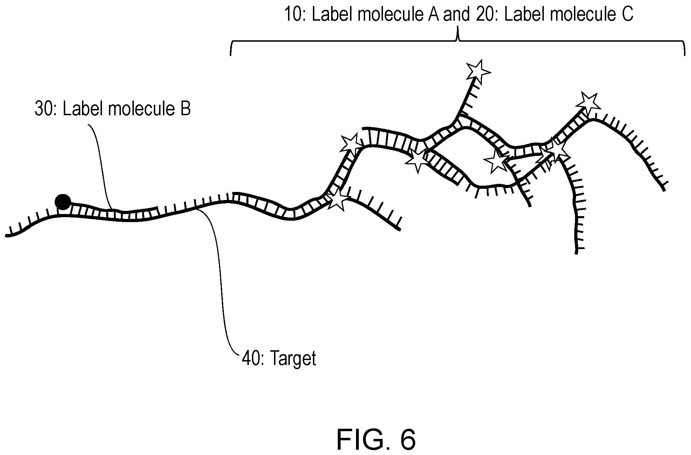

Fig. 6 schematically shows a complex which may be formed in one or more embodiments of the present disclosure. The complex comprises a target, a label molecule A, a label molecule B, and a label molecule C. In the complex shown in Fig. 6, the label molecule A is hybridized to a part of a target, the label molecule B is hybridized to another part of the target, and the label molecule C is hybridized to the label molecule A. In addition, another label molecule A is hybridized to the label molecule C, and another label molecule C is further hybridized thereto. That is, the complex comprises a target, a plurality of label molecules A, a label molecule B, and a plurality of label molecules C. The complex according to the present disclosure comprises a label molecule B, and, accordingly, it can be easily collected with the aid of a magnet or via precipitation. In addition, the complex has a plurality of phosphors derived from the label molecule A and the label molecule C reacting with a target, and high brightness is thus observed in fluorescence observation. In the method for producing a complex according to the present disclosure, accordingly, a complex can be easily observed even if a target is present at low concentration in the sample.

As described above, the step (a) involves the use of a label molecule A, a label molecule B, and a label molecule C as label molecules. The step (a) may further involve the use of other label molecules. In one or more embodiments involving the use of other label molecules, the step (a) may comprise bringing a sample containing a target into contact with a label molecule A, a label molecule B, a label molecule C, and a label molecule D and forming a complex comprising the target, the label molecule A, the label molecule B, the label molecule C, and the label molecule D. In such embodiment, the label molecule D comprises a nucleic acid sequence capable of hybridizing to a part of the label molecule B and substantially complementary to the nucleic acid sequence of the label molecule B. In such embodiment, at least one of either the label molecule B or the label molecule D comprises at least one of either magnetic microparticles or metal microparticles. In such embodiment, specifically, the complex comprises a target, a label molecule A hybridized to a part of the target, a label molecule B hybridized to another part of the target, a label molecule C hybridized to a part of the label molecule A, and a label molecule D hybridized to a part of the label molecule B.

In one or more embodiments involving the use of the label molecule D, at least one of either the label molecule B or the label molecule D comprises at least one of either magnetic microparticles or metal microparticles. In one or more embodiments, both the label molecule B and the label molecule D may comprise at least one of either magnetic microparticles or metal microparticles, and both the label molecule B and the label molecule D may comprise magnetic microparticles. At least one of either magnetic microparticles or metal microparticles may be located in any positions in the label molecule B or the label molecule D. For example, microparticles may be located at the molecular end (e.g., the 5' end or the 3' end). In one or more embodiments, specifically, the label molecule B may comprise at least one of either magnetic microparticles or metal microparticles at the molecular end, and the label molecule D may comprise at least one of either magnetic microparticles or metal microparticles at the molecular end.

In one or more embodiments involving the use of the label molecule D, the label molecule B may comprise an u region, a v region, and a w region, the label molecule D may comprise an U region and a V region, the u region may comprise a nucleic acid sequence substantially complementary to the nucleic acid sequence of the U region, the v region may comprise a nucleic acid sequence substantially complementary to the nucleic acid sequence of the V region, and the w region may comprise a nucleic acid sequence capable of hybridizing to a part of the target and substantially complementary to the nucleic acid sequence of the target. The label molecule B may comprise a nucleic acid sequence other than the nucleic acid sequence of the u region, the v region, or the w region, and the label molecule D may comprise a nucleic acid sequence other than the nucleic acid sequence of the U region or the V region.

In one or more embodiments involving the use of the label molecule D, examples of specific combinations of the label molecule B and the label molecule D include the following embodiments: one or more embodiments in which the label molecule B has two or more of at least either u regions or v regions in its molecule and the label molecule D has an U region and a V region in its molecule; one or more embodiments in which the label molecule D has two or more of at least either U regions and V regions in its molecule and the label molecule B has an u region and a v region in its molecule; and one or more embodiments in which the label molecule B has two or more of at least either u regions or v regions in its molecule and the label molecule D has two or more of at least either U regions and V regions in its molecule.

In one or more embodiments involving the use of the label molecule D, the label molecule B may comprise the w region comprising a nucleic acid sequence different from the nucleic acid sequence of the u region or the v region, and the nucleic acid sequence of at least either the u region or the v region may comprise the nucleic acid sequence of the w region

In one or more embodiments involving the use of the label molecule D, a complex would comprise a plurality of label molecules B and a plurality of label molecules D. This may facilitate collection with the aid of a magnet or collection via precipitation.

In one or more embodiments, the step (a) may comprise bringing a sample containing a target into contact with a label molecule A, a label molecule B, and a label molecule C. The step (a) may be performed by any method without particular limitation. For example, the label molecule A, the label molecule B, the label molecule C, and, according to need, a label molecule D may be simultaneously or successively added to the sample. Alternatively, the sample may be added to a solution containing the label molecule A, the label molecule B, the label molecule C, and, according to need, the label molecule D. A sample is derived from, for example, a test object. In a process of sample preparation, the sample may be supplemented with a solvent. An example of a solvent that can be used is a buffer or an aqueous solution containing salt such as sodium. Examples of buffers that can be used include saline sodium citrate (SSC) buffer, Church's phosphate buffer, and saline-sodium phosphate-EDTA (SSPE) buffer. When a sample prepared by subjecting a test object to the step (1) described below is used, the sample may be supplemented with a solvent to a concentration 2 to 10 times the final concentration (2-to 10-fold) of the test object subjected to the step (1) (i.e., the nucleic acid extract). In case of successive addition, the order of addition is not particularly limited. The step (a) may be performed in the air or in an inert gas atmosphere. The step (a) may be performed under ordinary pressure, under pressure, or under reduced pressure. The step (a) may be performed under ordinary pressure from the viewpoint of ease of operation. The step (a) may be performed at any temperature, provided that hybridization can take place. Such temperature is not particularly limited, it may be 30°C to 70°C. In one or more embodiments, such temperature may be 55°C to 65°C. A duration of the step (a) is not particularly limited, and it may be 15 to 240 minutes. In one or more embodiments, such duration may be 30 to 60 minutes.

In one or more embodiments, a step (b) is not particularly limited, provided that the complex formed in the step (a) can be collected. When the label molecule B or the label molecule D comprising magnetic microparticles is used, the complex may be collected with the aid of a magnet. When the label molecule B or the label molecule D comprising metal microparticles is used, the complex may be precipitated, the supernatant may be removed, and the complex may then be collected.

### (The method for determining microbial inclusion)

The method for determining microbial inclusion of the present disclosure comprises determining microbial inclusion in a test object, and the method comprises:
a step (1) of obtaining a sample by treatment of a test object under conditions in which a nucleic acid derived from the microorganism is extracted when the test object includes the microorganism;
a step (2) of bringing the sample into contact with a label molecule A, a label molecule B, and a label molecule C and, when the sample contains a nucleic acid derived from the microorganism, forming a complex comprising the target, the label molecule A, the label molecule B, and the label molecule C; and
a step (3) of isolating a complex when the complex is formed.

The target is a nucleic acid derived from the microorganism. In general, such nucleic acid comprises a particular sequence. The label molecule A comprises a nucleic acid sequence capable of hybridizing to a part of the target and substantially complementary to the nucleic acid sequence of the target. The label molecule B comprises a nucleic acid sequence capable of hybridizing to a part of the target and substantially complementary to the nucleic acid sequence of the target. The substantially complementary nucleic acid sequence of the label molecule A is capable of hybridizing to a part of the target and the substantially complementary nucleic acid sequence of the label molecule B is capable of hybridizing to another part of the target. The label molecule C comprises a nucleic acid sequence capable of hybridizing to a part of the label molecule A and substantially complementary to the nucleic acid sequence of the label molecule A. At least one of either the label molecule A or the label molecule C has a phosphor.

According to the method for determining microbial inclusion of the present disclosure, microbial inclusion can be determined in a rapid manner, a procedure is simple, and use of an expensive reagent or enzyme is not necessary. Thus, the testing cost can be reduced.

Test objects are required to be evaluated as to microbial inclusion therein. Examples thereof include food, pharmaceutical, cosmetic, and daily-use products. In a test object without any processing, in general, a target nucleic acid cannot be detected. Accordingly, the test object is subjected to treatment under conditions in which a nucleic acid derived from a microorganism is extracted when the test object includes the microorganism, and a product of the treatment is used as the sample.

According to the method for determining microbial inclusion described above, the test object is determined to include microorganisms when a complex is obtained with the use of a microbe-derived nucleic acid sequence as a target; in other words, when high brightness is observed in fluorescence observation. In general, it is highly unlikely that a test object includes microorganisms. Accordingly, a simple method is desired for testing microbial inclusion. In one or more embodiments, accordingly, a nucleic acid sequence that is common among as many microorganisms as possible may be employed as a sequence to which the label molecule A or the label molecule B is hybridized. Thus, microbial inclusion can be determined through a small number of testing instances or with the use of a small number of types of label molecules A or label molecules B. When inclusion of a particular microorganism is to be determined, in contrast, a sequence peculiar to a particular microorganism may be employed as a sequence to which the label molecule A or the label molecule B is hybridized. Thus, inclusion of a particular microorganism can be determined.

When the test object includes the microorganism, the step (2) in one or more embodiments is substantially the same step as the step (a) in the method for producing a complex described above, and the step (3) in one or more embodiments is substantially the same step as the step (b) in the method for producing a complex described above. In one or more embodiments, more specifically, the label molecule B may comprise at least one of either magnetic microparticles or metal microparticles as described above. While the step (a) involves the use of the label molecule A, the label molecule B, and the label molecule C, the step (a) may be performed with the use of additional other label molecules. In one or more embodiments involving the use of other label molecules, for example, the step (2) described above may comprise bringing a sample into contact with a label molecule A, a label molecule B, a label molecule C, and a label molecule D and, when the sample contains a nucleic acid derived from the microorganism, forming a complex comprising the target, the label molecule A, the label molecule B, the label molecule C, and the label molecule D. According to such embodiment, the label molecule D comprises a nucleic acid sequence capable of hybridizing to a part of the label molecule B and substantially complementary to the nucleic acid sequence of the label molecule B. According to such embodiment, at least one of either the label molecule B or the label molecule D has at least one of either magnetic microparticles or metal microparticles. When the test object does not include the microorganism, a complex would not be formed in the step (2). When the test object does not include the microorganism, accordingly, the complex would not be isolated in the step (3). In such a case, the label molecule B or the label molecule D, which may be optionally used, would be selectively collected in the step (3). In one or more embodiments, accordingly, the collected complex would exhibit high brightness in fluorescence observation when the test object includes the microorganism. This enables determination of microbial inclusion. In one or more embodiments, in addition, the collected label molecule B or the label molecule D, which may be optionally used, would not exhibit high brightness in fluorescence observation when the test object does not include the microorganism. Accordingly, it would be possible to determine that the test object does not include the microorganism.

In one or more embodiments, the step (1) may comprise treating a test object under conditions in which a nucleic acid derived from the microorganism is extracted when the test object includes the microorganism, and details of the step (1) are not particularly limited. An example of the step (1) that can be adopted is a method of high-temperature, high-pressure extraction, which is known as a method for nucleic acid extraction from bacteria. In one or more embodiments, the step (1) may comprise a step of introducing a test object into a container, a step of hermetically sealing the container, and a step of heating the test object hermetically sealed in the container to 100°C or higher in a hermetically sealed state. In one or more embodiments, the temperature may be 100°C to 180°C. In some other embodiments, the temperature may be 130°C to 160°C.

The sample obtained in the step (1) may be subjected to the step (2) without any processing. Alternatively, a step of eliminating contaminants from the sample or other steps may be performed between the step (1) and the step (2).

### (The method for identifying the included microorganism)

The method for identifying the included microorganism according to the present disclosure comprises identifying the microorganism included in a test object, and the method comprises:
a step (2) of bringing a sample derived from the test object into contact with a label molecule A, a label molecule B, and a label molecule C and, when the sample contains a nucleic acid derived from the microorganism, forming a complex comprising the target, the label molecule A, the label molecule B, and the label molecule C;
a step (3) of isolating a complex when the complex is formed; and
a step (4) of identifying the microorganism from which the nucleic acid sequence of the target contained in the complex is derived.

The target is a nucleic acid derived from the microorganism. In general, such nucleic acid comprises a particular sequence. The label molecule A comprises a nucleic acid sequence capable of hybridizing to a part of the target and substantially complementary to the nucleic acid sequence of the target. The label molecule B comprises a nucleic acid sequence capable of hybridizing to a part of the target and substantially complementary to the nucleic acid sequence of the target. The substantially complementary nucleic acid sequence of the label molecule A is capable of hybridizing to a part of the target and the substantially complementary nucleic acid sequence of the label molecule B is capable of hybridizing to another part of the target. The label molecule C comprises a nucleic acid sequence capable of hybridizing to a part of the label molecule A and substantially complementary to the nucleic acid sequence of the label molecule A. At least one of either the label molecule A or the label molecule C has a phosphor.

The step (2) and the step (3) in one or more embodiments are the same as the step (2) and the step (3) in the method for determining microbial inclusion described above. In one or more embodiments, the step (1) described with regard to the method for determining microbial inclusion described above may be performed before the step (2). When the step (1) is performed in one or more embodiments, as with the case of the method for determining microbial inclusion described above, the sample obtained in the step (1) may be subjected to the step (2) without any processing. Alternatively, a step of eliminating contaminants from the sample or other steps may be performed between the step (1) and the step (2).

As described with regard to the method for determining microbial inclusion described above, a nucleic acid sequence that is common among as many microorganisms as possible may be employed as a sequence to which the label molecule A or the label molecule B is hybridized. When the complex is isolated in the step (3), accordingly, it is generally difficult to specifically identify the included microorganism. In the method for identifying the included microorganism according to the present disclosure, the step (4) enables identification of the microorganism from which the nucleic acid sequence of the target is derived. Thus, the included microorganism can be identified.

The step (4) is performed when the complex is isolated in the step (3). In the step (4), the microorganism from which the nucleic acid sequence of the target is derived may be identified. A specific means of performing the step (4) is not particularly limited. Examples of means that can be adopted include the microarray technique, PCR, and sequencing. The microarray technique may be performed with the use of signaling arrays, or a technique involving the use of self-assembling labels for microarray detection may be employed. The method involving the use of signaling arrays may be performed in accordance with, for example, JP 2015-043702 A. An example of the step (4) comprises: suspending a complex in an NaOH solution of approximately 1 N (e.g., 0.5 to 2 N) again; collecting the label molecule B or the label molecule D with the aid of a magnet or via centrifugation again; neutralizing the solution with HCl of approximately 1 N (e.g., 0.5 to 2 N); adding a buffer (e.g., SSC buffer) to adjust the final concentration to approximately 2 to 10 times (2- to 10-fold); detecting using microarrays comprising signaling array probes immobilized thereon; and identifying the bacterial species or detecting a particular gene. Instead of the use of an NaOH solution, the complex may be heated to, for example, approximately 95°C (i.e., 90°C to 100°C), so as to dissociate the label molecules A to D from the target. When the amount of the target is small, nucleic acids may be amplified via PCR or other means, and detection may then be performed using microarrays or the like.

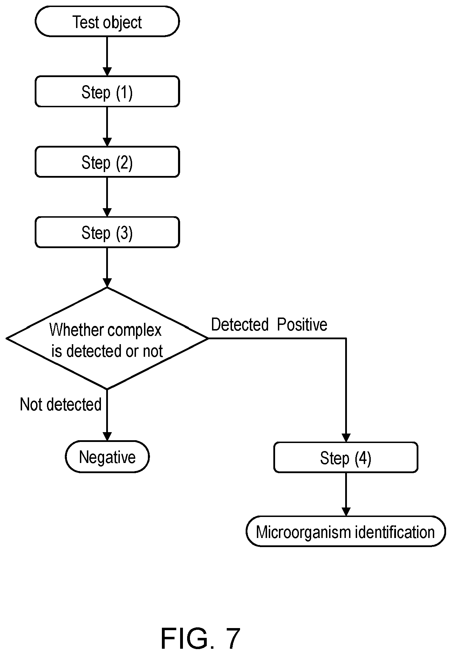

Fig. 7 shows one or more embodiments of the method for identifying the included microorganism comprising the step (1) to the step (4). In one or more embodiments shown in Fig. 7, the step (1) to the step (3) are performed, and the presence or absence of the complex is examined by, for example, fluorescence observation. When fluorescence is observed, the complex is detected; i.e., the detection is determined positive, and the step (4) is performed to identify the included microorganism. When fluorescence is not observed, the complex is not detected; i.e., the detection is determined negative, and the procedure is completed.

### Examples

Hereafter, the present disclosure is described with reference to the examples, although the present disclosure is not limited to these examples.

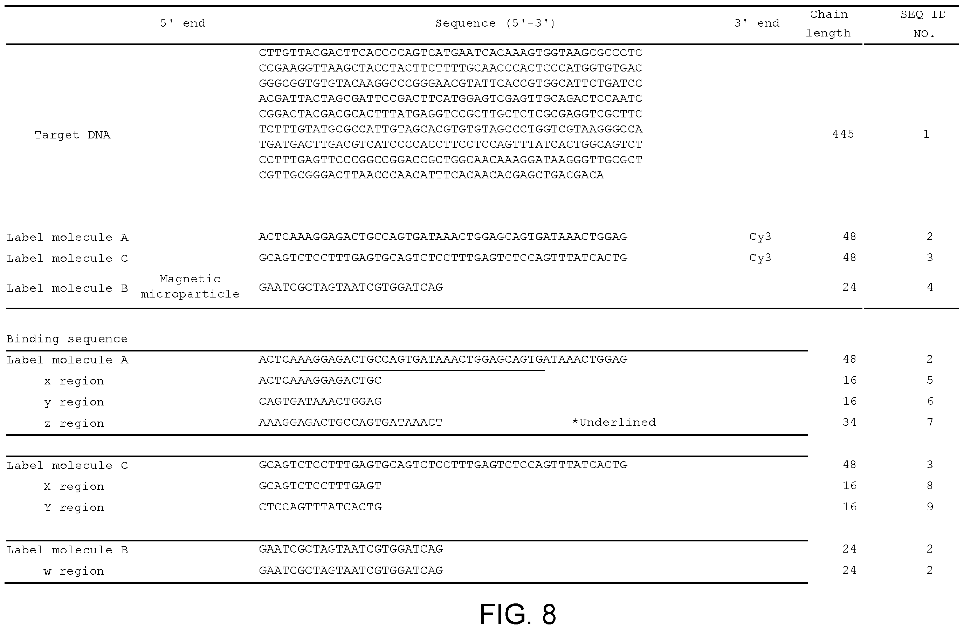

The sequence of the synthetic oligo DNA (445 nt) (a target DNA) used in the example and the comparative example is shown in SEQ ID NO: 1 in the sequence listing, the sequence of the label molecule A is shown in SEQ ID NO: 2 in the sequence listing, the sequence of the label molecule C is shown in SEQ ID NO: 3 in the sequence listing, and the sequence of the label molecule B is shown in SEQ ID NO: 4 in the sequence listing. The sequence of the x region of the label molecule A used in the example and the comparative example is shown in SEQ ID NO: 5 in the sequence listing, the sequence of the y region is shown in SEQ ID NO: 6 in the sequence listing, the sequence of the z region is shown in SEQ ID NO: 7 in the sequence listing, the sequence of the X region of the label molecule C is shown in SEQ ID NO: 8 in the sequence listing, and the sequence of the Y region is shown in SEQ ID NO: 9 in the sequence listing. The sequence of the w region of the label molecule B is the same as the sequence of the label molecule B. These sequences are also shown in Fig. 8.

Synthesis of the synthetic oligo DNA was outsourced to Eurofins Genomics and the synthetic oligo DNA was obtained therefrom.

Concerning the label molecule A, DNA synthesis and 3' end modification with a fluorescent molecule (Cy3) were outsourced to Eurofins Genomics, and the label molecule A was obtained therefrom. The label molecule A used in the example has the structure schematically shown in Fig. 3; i.e., the label molecule A comprises the nucleic acid sequences of the x region, the y (first) region, and the (second) y region from the 5' end and comprises a phosphor at the 3' end.

Concerning the label molecule C, DNA synthesis and 3' end modification with a fluorescent molecule (Cy3) were outsourced to Eurofins Genomics, and the label molecule C was obtained therefrom. The label molecule C used in the example has the structure schematically shown in Fig. 4; i.e., the label molecule C comprises the nucleic acid sequences of the (first) X region, the (second) X region, and the Y region from the 5' end and comprises a phosphor at the 3' end.

Concerning the label molecule B, DNA synthesis and 5' end modification with an amino group were outsourced Eurofins Genomics, NHS magnetic beads (Thermo Scientific) were bound to the amino group of the obtained amino-modified DNA, and the label molecule B was thus obtained. NHS magnetic beads were bound to the aforementioned amino group in accordance with the instructions of the magnetic beads product. The label molecule B used in the example has the structure schematically shown in Fig. 5; i.e., the label molecule B comprises the nucleic acid sequence of the w region and magnetic microparticles at the 5' end.

### [Comparative Example 1]

The synthetic oligo DNA comprising the E. coli 16S rDNA sequence (445 nt) was employed as a target. The synthetic oligo DNA was diluted with DW (deionized distilled water) to adjust the final concentration to 1 nM, and a target solution was obtained.

The target solution, the label molecule B (final concentration: 100 nM), and the label molecule A (final concentration: 100 nM) were incubated in a 5-fold diluted solution of SSC buffer (total volume: 40 µl) at 60°C for 30 minutes, and particles (the complex comprising the target, the label molecule A, and the label molecule B) were collected using a magnet.

The particles were washed 3 times with SSC buffer (40 µl). SSC buffer (40 µl) was added again, the particles were converged using a magnet, and fluorescence of the particles was observed by applying a laser beam at 532 nm. Fluorescence observation was performed using a CCD (charge-coupled device) detector, the DPSS (diode-pumped solid-state) light source at 532 nm, glass cells (6 × 8 × 1 mm), and a common optical system for fluorescence observation.

Since particles were converged with the use of a magnet, the particles were considered to comprise the label molecule B. Since fluorescence was observed, the particles were considered to comprise the label molecule A. Since the label molecule B would not directly bind (hybridize) to the label molecule A, the particles were verified to be a complex comprising the target, the label molecule A, and the label molecule B.

As a negative control, similar testing was performed without the addition of the target molecules. Substantially no fluorescence was observed in the negative control, which did not involve the use of the target.

### [Example 1]

The synthetic oligo DNA comprising the E. coli 16S rDNA sequence (445 nt) was employed as a target. The synthetic oligo DNA was diluted with DW (deionized distilled water) to adjust the final concentration to 1 nM, and a target solution was obtained.

The target solution, the label molecule B (final concentration: 100 nM), the label molecule A (final concentration: 100 nM), and the label molecule C (final concentration: 100 nM) were incubated in a 5-fold diluted solution of SSC buffer (total volume: 40 µl) at 60°C for 30 minutes, and particles (the complex comprising the target, the label molecule A, the label molecule B, and the label molecule C) were collected using a magnet.

The particles were washed 3 times with SSC buffer (40 µl). SSC buffer (40 µl) was added again, the particles were converged using a magnet, and fluorescence of the particles was observed by applying a laser beam at 532 nm.

Since particles were converged with the use of a magnet, the particles were considered to comprise the label molecule B. Since fluorescence was observed at a higher level than Comparative Example 1, the particles were considered to comprise the label molecule A and the label molecule C. Since the label molecule B would not directly bind (hybridize) to the label molecule A or C, the particles were verified to be a complex comprising the target, the label molecule A, the label molecule B, and the label molecule C.

As a negative control, similar testing was performed without the addition of the target. Substantially no fluorescence was observed in the negative control, which did not involve the use of the target.

Table 1 shows the results of fluorescence observation in Comparative Example 1, the results of fluorescence observation in Example 1, and the results of fluorescence observation of the negative control in Example 1.

**Table 1**

| | Example 1 Negative control | Comparative Example 1 | Example 1 |
|---|---|---|---|
| Detection intensity (a.u.) | 1,062 | 7,240 | 29,258 |
| Standard deviation (a.u.) | 62 | 242 | 1,014 |

The upper limits and/or the lower limits of the numerical ranges described herein may be employed in any combination. For example, a numerical range may be defined by employing an upper limit and a lower limit in any combination, a numerical range may be defined by employing upper limits in any combination, or a numerical range may be defined by employing lower limits in any combination. In addition, the numerical ranges expressed with the use of the preposition "to" used herein encompass the values before and after the preposition "to" as the lower limit and the upper limit.

The present disclosure was described in detail above. It should be noted that the specific constitutions are not limited to the embodiments described above and that design modification and various other embodiments performed within the scope of the present disclosure as determined by the claims are included in the present disclosure.

### DESCRIPTION OF SYMBOLS

10: Label molecule A
11: x region
13: y region
15: z region
17: Phosphor
20: Label molecule C
21: X region
23: Y region
30: Label molecule B
31: w region
33: Magnetic microparticle
40: Target

## Claims

1. A method for producing a complex comprising a target (40) that is contained in a sample, the method comprising:
A step (a) of bringing a sample containing the target (40) into contact with a label molecule A (10), a label molecule B (30), and a label molecule C (20) and forming a complex comprising the target (40), the label molecule A (10), the label molecule B (30), and the label molecule C (20); and
a step (b) of isolating the complex,
wherein
the target (40) is a nucleic acid,
the label molecule A (10) comprises a nucleic acid sequence capable of hybridizing to a part of the target (40) and substantially complementary to a nucleic acid sequence of the target (40),
the label molecule B (30) comprises the nucleic acid sequence capable of hybridizing to a part of the target (40) and substantially complementary to the nucleic acid sequence of the target (40),
a substantially complementary nucleic acid sequence of the label molecule A (10) is capable of hybridizing to a part of the target (40) and a substantially complementary nucleic acid sequence of the label molecule B (30) is capable of hybridizing to another part of the target (40),
the label molecule C (20) comprises a nucleic acid sequence capable of hybridizing to a part of the label molecule A (10) and substantially complementary to the nucleic acid sequence of the label molecule A (10), and
at least one of either the label molecule A (10) or the label molecule C (20) includes a phosphor (17).

2. The method for producing a complex according to claim 1, wherein the label molecule B (30) includes at least one of either magnetic microparticles (33) or metal microparticles.

3. The method for producing a complex according to claim 1, wherein
the step (a) comprises:
bringing the sample containing the target (40) into contact with the label molecule A (10), the label molecule B (30), the label molecule C (20), and a label molecule D; and
forming the complex comprising the target (40), the label molecule A (10), the label molecule B (30), the label molecule C (20), and the label molecule D,
the label molecule D includes a nucleic acid sequence capable of hybridizing to a part of the label molecule B (30) and substantially complementary to the nucleic acid sequence of the label molecule B (30), and
at least one of either the label molecule B (30) or the label molecule D includes at least one of either magnetic microparticles (33) or metal microparticles.

4. The method for producing a complex according to any one of claims 1 to 3, wherein
the label molecule A (10) includes an x region (11), a y region (13), and a z region (15),
the label molecule C (20) includes an X region (21) and a Y region (23),
the x region (11) is a nucleic acid sequence substantially complementary to a nucleic acid sequence of the X region (21),
the y region (13) is a nucleic acid sequence substantially complementary to a nucleic acid sequence of the Y region (23), and
the z region (15) is a nucleic acid sequence capable of hybridizing to a part of the target (40) and substantially complementary to the nucleic acid sequence of the target (40).

5. The method for producing a complex according to claim 4, wherein
the label molecule A (10) includes two or more of at least either x regions (11) or y regions (13) in a molecule of the label molecule A (10) and the label molecule C (20) includes an X region (21) and a Y region (23) in a molecule of the label molecule C (20),
the label molecule C (20) includes two or more of at least either X regions (21) or Y regions (23) in a molecule of the label molecule C (20) and the label molecule A (10) includes an x region (11) and a y region (13) in a molecule of the label molecule A (10), or
the label molecule A (10) includes two or more of at least either x regions (11) or y regions (13) in a molecule of the label molecule A (10) and the label molecule C (20) includes two or more of at least either X regions (21) and Y regions (23) in a molecule of the label molecule C (20).

6. The method for producing a complex according to claim 3, wherein
the label molecule B (30) includes an u region, a v region, and a w region (31),
the label molecule D includes an U region and a V region,
the u region is a nucleic acid sequence substantially complementary to a nucleic acid sequence of the U region,
the v region is a nucleic acid sequence substantially complementary to a nucleic acid sequence of the V region, and
the w region (31) is a nucleic acid sequence capable of hybridizing to a part of the target (40) and substantially complementary to the nucleic acid sequence of the target (40).

7. The method for producing a complex according to claim 6, wherein
the label molecule B (30) includes two or more of at least either u regions or v regions in a molecule of the label molecule B (30) and the label molecule D includes an U region and a V region in a molecule of the label molecule D,
the label molecule D includes two or more of at least either U regions or V regions in a molecule of the label molecule D and the label molecule B (30) includes an u region and a v region in a molecule of the label molecule B (30), or
the label molecule B (30) includes two or more of at least either u regions or v regions in a molecule of the label molecule B (30) and the label molecule D includes two or more of at least either U regions or V regions in a molecule of the label molecule D.

8. A method for determining microbial inclusion in a test object, the method comprising:
a step (1) of obtaining a sample by treatment of a test object under conditions in which a nucleic acid derived from a microorganism is extracted when the test object includes the microorganism;
a step (2) of bringing the sample into contact with a label molecule A (10), a label molecule B (30), and a label molecule C (20) and, when the sample contains a nucleic acid derived from the microorganism, forming a complex comprising a target (40), the label molecule A (10), the label molecule B (30), and the label molecule C (20); and
a step (3) of isolating the complex when the complex is formed,
wherein
the target (40) is a nucleic acid derived from the microorganism,
the label molecule A (10) comprises a nucleic acid sequence capable of hybridizing to a part of the target (40) and substantially complementary to a nucleic acid sequence of the target (40),
the label molecule B (30) comprises the nucleic acid sequence capable of hybridizing to a part of the target (40) and substantially complementary to the nucleic acid sequence of the target (40),
a substantially complementary nucleic acid sequence of the label molecule A (10) is capable of hybridizing to a part of the target (40) and a substantially complementary nucleic acid sequence of the label molecule B (30) is capable of hybridizing to another part of the target (40),
the label molecule C (20) comprises a nucleic acid sequence capable of hybridizing to a part of the label molecule A (10) and substantially complementary to the nucleic acid sequence of the label molecule A (10), and
at least one of either the label molecule A (10) or the label molecule C (20) includes a phosphor (17).

9. The method for determining microbial inclusion according to claim 8, wherein the label molecule B (30) includes at least one of either magnetic microparticles (33) or metal microparticles.

10. The method for determining microbial inclusion according to claim 8, wherein the step (2) comprises:
bringing the sample into contact with the label molecule A (10), the label molecule B (30), the label molecule C (20), and a label molecule D; and
when the sample contains the nucleic acid derived from the microorganism, forming a complex comprising the target (40), the label molecule A (10), the label molecule B (30), the label molecule C (20), and the label molecule D,
the label molecule D includes a nucleic acid sequence capable of hybridizing to a part of the label molecule B (30) and substantially complementary to the nucleic acid sequence of the label molecule B (30), and
at least one of either the label molecule B (30) or the label molecule D includes at least one of either magnetic microparticles (33) or metal microparticles.

11. The method for determining microbial inclusion according to any one of claims 8 to 10, wherein the step (1) comprises:
a step of introducing the test object into a container;
a step of hermetically sealing the container; and
a step of heating the test object hermetically sealed in the container to 100°C or higher in a hermetically sealed state.

12. A method for identifying a microorganism included in a test object, the method comprising:
a step (2) of bringing a sample derived from the test object into contact with a label molecule A (10), a label molecule B (30), and a label molecule C (20) and, when the sample contains a nucleic acid derived from the microorganism, forming a complex comprising a target (40), the label molecule A (10), the label molecule B (30), and the label molecule C (20);
a step (3) of isolating the complex when the complex is formed; and
a step (4) of identifying the microorganism from which a nucleic acid sequence of the target (40) contained in the complex is derived,
wherein
the target (40) is a nucleic acid derived from the microorganism,
the label molecule A (10) comprises a nucleic acid sequence capable of hybridizing to a part of the target (40) and substantially complementary to the nucleic acid sequence of the target (40),
the label molecule B (30) comprises a nucleic acid sequence capable of hybridizing to a part of the target (40) and substantially complementary to the nucleic acid sequence of the target (40),
a substantially complementary nucleic acid sequence of the label molecule A (10) is capable of hybridizing to a part of the target (40) and a substantially complementary nucleic acid sequence of the label molecule B (30) is capable of hybridizing to another part of the target (40),
the label molecule C (20) comprises a nucleic acid sequence capable of hybridizing to a part of the label molecule A (10) and substantially complementary to the nucleic acid sequence of the label molecule A (10), and
at least one of either the label molecule A (10) or the label molecule C (20) includes a phosphor (17).

13. The method for identifying the included microorganism according to Claim 12, wherein the label molecule B (30)includes at least one of either magnetic microparticles (33) or metal microparticles.

14. The method for identifying the included microorganism according to claim 12, wherein the step (2) comprises:
bringing a sample derived from a test object into contact with the label molecule A (10), the label molecule B (30), the label molecule C (20), and a label molecule D; and
when the sample contains a nucleic acid derived from the microorganism, forming a complex comprising the target (40), the label molecule A (10), the label molecule B (30), the label molecule C (20), and the label molecule D,
the label molecule D includes a nucleic acid sequence capable of hybridizing to a part of the label molecule B (30) and substantially complementary to the nucleic acid sequence of the label molecule B (30), and
at least one of either the label molecule B (30) or the label molecule D includes at least one of either magnetic microparticles (33) or metal microparticles.

15. The method for identifying the included microorganism according to any one of claims 12 to 14, which comprises, before the step (2):
a step (1) of obtaining a sample by treatment of the test object under conditions in which the nucleic acid derived from the microorganism is extracted when the test object includes the microorganism.
